# EUROPEAN PATENT APPLICATION

(11) **EP 1 016 452 A2**
(43) Date of publication of application: **05.07.2000**
(21) Application number: 00101314.3
(22) Date of filing: 12.11.1996
(51) Int. Cl.: B01F 13/00, B01F 13/06, A61F 2/46

(54) **Method and device for mixing components for bone cement in a mixing vessel**

(30) Priority: 13.11.1995 US 545591; 22.10.1996 US 734817
(62) Divisional of application: 96939409.7
(71) Applicant: CEMVAC SYSTEM AKTIEBOLAG, 589 41 Linköping (SE)
(72) Inventor: Jonsson, Sören, 589 41 Linköping (SE)
(74) Representative: Willquist, Bo

(57) **Abstract**

An apparatus for successively feeding batches of a liquid component (A) into a mixing vessel (2) maintained under vacuum for preparation of a bone cement in order to prevent harmful emissions from escaping from said vessel (2) during feeding of component (A) and during mixing of the liquid and powder components (A, B). The mixing vessel (2) is pre-filled with the powder component (B). The vessel (2) is defined by an outer wall (3) having a first end and a second end. One of said first or second ends is formed with a sealable spout (5), the other is formed with an axially displaceable bottom (80), the bottom having a central opening therein. An agitator (6) is received within the vessel and comprises a tubular rod (6b) which extends upwardly through the opening in the bottom (80) and an agitator disk (6b) attached to the tubular rod. The tubular rod (6b) has an open, first end that defines a mouth and an open, second end that is encircled by the disk, and is axially displaceable within the vessel for mixing the bone cement components (A, B). The apparatus comprises a means for holding and introducing component (A) into the mixing vessel (2) being joined to the mouth of the tubular rod prior to feeding component (A) into the vessel (2) through the tubular rod (6b). The powder and liquid components (B, A) are mixed in the vessel interior (2a) under a continuous vacuum whereafter the mixed bone cement is pushed out through the sealable spout (5) through a displacement of the vessel bottom (80).

## Description

### BACKGROUND OF THE INVENTION

### Field of the Invention

The present invention relates to a method for mixing constituent components in a mixing vessel for the preparation of bone cement under vacuum. The invention also relates to an apparatus for mixing constituent components in a mixing vessel under partial vacuum for the preparation of bone cement.

### Description of the Prior Art

Bone cement is prepared by mixing polymethyl methacrylate in powder form, with liquid monomethyl methacrylate in a mixing container. Both the liquid component and the combined mixture give off substances in gaseous form which are environmentally harmful and injurious to the health. For this reason, it is important for the introduction of the bone cement components into the mixing container and the mixing process itself, to take place in such a way that the smallest possible quantity of the harmful gases escape into the surrounding environment. Mixing vessels in which the introduced components were successfully prepared into bone cement without a substantial release of the aforementioned gases are described in SE-C-8901599-4 and SE-A0-9201353-1 and WO 94/26403, for example. In order for the bone cement to develop optimal strength during use, it is also important for the components comprising the cement to have well-mixed, predetermined portions.

In said WO-publication a glass ampoule containing a liquid component of a bone cement is surrounded by a container which is in reclosable communication with the atmosphere. A second container surrounds the first container so that when the mixing vessel is opened, the contents of the ampoule, under the effect of a partial vacuum inside the mixing vessel, can be sucked down into it, a space formed between the aforementioned inner container and outer container is filled with a second bone cement component in powder form which is caused by displacement of the inner container relative to the outer container, to move from a first position in which the space does not communicate with the atmosphere or the mixing vessel to a second position in which the space communicates with the atmosphere and the mixing vessel, so that the powdered bone cement component is sucked down into the mixing vessel under the effect of the partial vacuum inside it.

A device for carrying out the above method which device is also explained in said WO-publication is characterized in that it comprises, an inner container communicating with the atmosphere, and is so arranged as to enclose the glass ampoule containing the liquid bone cement component, and to communicate with the aforementioned mixing vessel, and which includes means for opening the ampoule so that its contents, under the effect of the partial vacuum inside the mixing vessel, can be sucked down into it. The outer container at least partially encloses the inner container and is also arranged so as to communicate with the mixing vessel and together with the inner container, defines a space therebetween which is filled with a certain quantity of the powdered component of bone cement. The inner container is capable of displacement from a first position to a second position, the first position characterized by the inner container preventing communication between both the mixing vessel and the atmosphere, and the second position characterized, in which communication between the mixing vessel on the one hand and the atmosphere on the other hand is open, so that the bone cement component in powder form, under the effect of the partial vacuum inside the mixing vessel, can be sucked into it without escape of gases.

### SUMMARY OF THE INVENTION

One object of the present invention is to make available a method and device of the kind described above, which avoids the risk of gas release when feeding the bone cement components into the mixing vessel. Another object is to make the mixing as convenient and simple as possible for a person using the device. Still another object is to make the mixing as safe as possible for said person i.e. reduce to a minimum the failure rate of mixing. This is achieved in accordance with the invention in that the mixing vessel is pre-filled with the powder bone cement and the first container holding the ampoule of the liquid bone cement component connected to the mixing vessel in a manner where displacement of a container cap causes the ampoule to break and the liquid component to be sucked into the mixing vessel under vacuum. The container is melded to the mixing tube for directly draining into the bottom of the mixing vessel.

The device for carrying out the method in accordance with this invention is more fully detailed in the following sections.

### BRIEF DESCRIPTION OF THE DRAWINGS

The invention is described below in greater detail with reference to the accompanying drawing, in which:

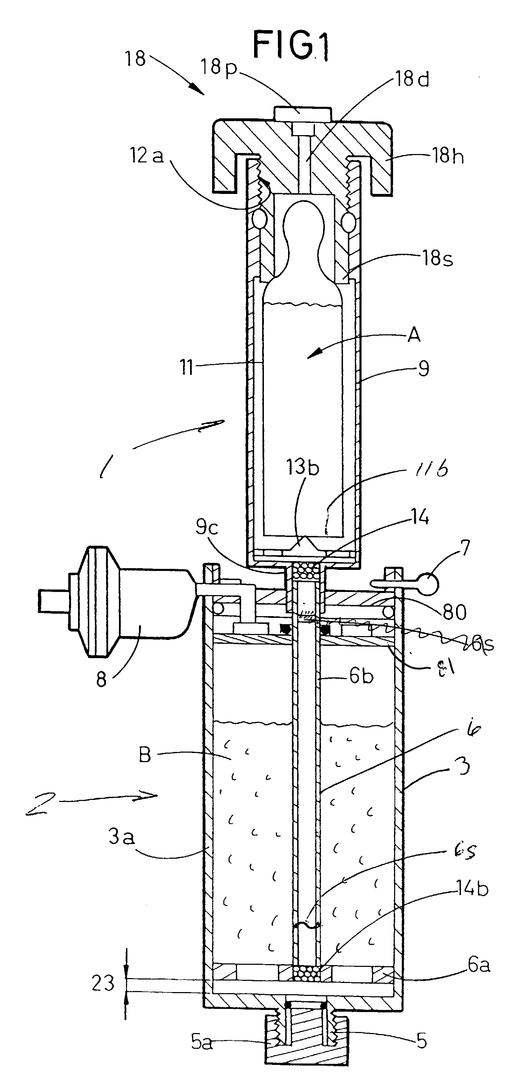

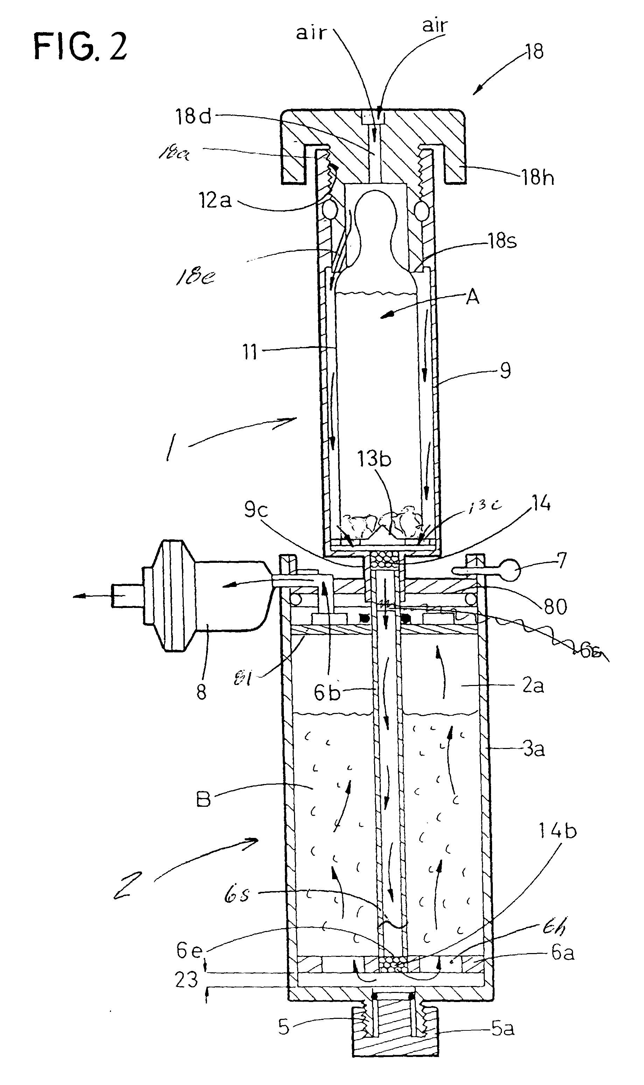

Figures 1 and 2 illustrate the mixing vessel of an embodiment of the present invention where a liquid cement container is inserted inside the tubular rod and joined thereto and the bone cement components are mixed.

### DETAILED DESCRIPTION OF THE INVENTION

The designations 1 and 2 are used generally in the drawing in respect of a feed arrangement and a mixing vessel. The latter comprises an interior 2a, a cylindrical container 3 comprised of an outer cylinder wall 3a, a bottom 80 forming a piston head at one end of the container and a spout 5 with sealed opening at the other end, together with an agitator 6, received within said spout and mixing vessel and capable of axial, vertical movement inside the container 3. The agitator 6 consists of an agitator disc 6a attached to a tubular agitator rod 6b. The agitator 6 is mounted so that it is free to vertically slide up and down while maintaining a seal in the spout 5, in such a way that the plurality of holes 6h in agitator disk 6a can be used to bring about through mixing of the bone cement components A, B within mixing vessel 2 with no gaseous escape to atmosphere. Once mixing is complete, and once a lock 7 has been removed, the bottom 80 can be axially displaced inside the cylinder by upward movement of piston head 80, moving towards the spout 5. The pistonlike function of bottom 80, upwardly pushes and then discharges the mixed bone cement via the spout 5, which now serves as a discharge nozzle. The interior of the container 2 communicates via a filter 8 with a vacuum source (not shown) during feeding and mixing of the bone cement components A, B. Rapid and effective feeding of the bone cement components A, B into the mixing vessel, and safe handling of the gases that are environmentally harmful and injurious to human health, are achieved in this way.

Figures 1 and 2 show in greater detail that glass ampoule tip 11a is pointing upwards when inserted within container 9, and that the ampoule is resting upon the upward cone 13b of breaking means, said means having internal passages 13c for allowing liquid there through once it passes filter 14 the object of which is to separate glass particles from the liquid component A. Cap 18 has gripping means 18h for facilitating the operative threading movement of cap 18 along container threads 12a. When handle 18h of the cap is turned so as to downwardly displace the cap 18 through action of the interacting threads 12a and 18a, shoulder 18s pushed downwardly against ampoule 11, causing upward-facing, pointed cone 13b to break bottom 11b of the ampoule, thereby allowing liquid contents A to flow through filter 14 under suction downwardly into hollow tube 6b as previously described. Opening 18d in cap 18, allow atmospheric air to be communicated into the interior of container 9 under suction, thereby preventing noxious fumes escaping to atmosphere. A small gap 23 exists between vessel bottom 4 and agitator disk 6a so that as liquid A descends tubular rod 6b, exists open end 6e, then it enters gap 23, which behaves as a passage for percolating an air/liquid mixture upwardly through holes 6h in the agitator disk 6a, so that air bubbles cause liquid A to thoroughly mix with the powder component B, while under the continuing action of the vacuum source.

Furthermore, the container 9 is formed with a neck 9c that either inserts inside tubular rod 6b or as shown here, is formed so that rod 6b inserts inside neck 9c. In either case, once the connection is made, the neck and the tube are joined together by known methods of friction spin-melting the like elastomeric components together, or they can be joined by conventional methods such as gluing, threading, or snap-fitting the pieces airtight to each other. Once connected, the cap 18 gripping means 18h are operatively threaded downward along container threads 12a, until shoulders 18s cause the glass ampoule to be broken against pointed cone 13b of the breaking means. The liquid component A passes from container 9, downwardly through filter 14, now located in neck 9c, then into hollow tubular member 6b, before passing through a second filter 14b at tube open end 6e. Ambient air is drawn together with the liquid A into the vessel thereby preventing fume to escape to the outside of the container. The liquid A then changes direction at the bottom of mixing vessel 2 due to the action of vacuum from vacuum source not shown and turns upwardly to percolate into the powdered bone cement component B premixing will then be performed of the cement components A and B. Plug 18p is then inserted into opening 18d to seal container 9 from atmospheric influence. The container 9 is gripped as a handle and stroked up and down, thereby causing agitator disk 6a to mix the two bone cement components A, B together, as previously explained with the other embodiments. As Figures 1 and 2 show, tubular element 6b is scored with an annular notch 6s, that allows the container-tubular rod to be separated from each other by snapping the container in a perpendicular direction to the rod, thereby breaking the rod at the notch. This step is performed after mixing is complete and after the tubular rod and agitator stick have been pulled back towards piston 80. Once the tubular element 6b is pulled back, and snapped off, mixing vessel 2 is turned upside down in order to facilitate removal of the mixed bone cement. Spout 5 is sealed with a removable closure 5a and where the cylindrical container outer wall 3a also acts as the mixing vessel bottom, wherein the agitator 6 is inserted through the bottom of the container 3. With this arrangement, once the mixing is completed and the rod fractured at notch 6s, pin 7 is removed, as is filter 8 to vacuum source, so that piston 80 is advanced into contact with the bone cement mixture and the mixture is pushed out of mixing vessel 2 through spout 5, of course once closure 5a is unthreaded and removed. A filter 81 resting against the piston 80 prevents cement from entering into the vacuum source and will likewise by means of filter 14b prevent cement from leaking out when the piston is advanced into contact with the cement. Spout 5 is used for attaching of various extrusion nozzles.

## Claims

1. An apparatus for successively feeding batches of a liquid component (A,) into an interior of a mixing vessel (2) for the preparation of a bone cement, said mixing vessel (2) interior (2a) maintained under a vacuum created from a vacuum source in order to prevent harmful emissions from escaping from said vessel (2) during feeding of a liquid (A) and once said liquid and powder components (A, B) are mixed, comprising:
a mixing vessel (2) pre-filled with a powder component (B) of said bone cement said vessel (2) defined by an outer wall (3) having a first end, a second end and an interior (2a), one of said first or second ends formed with a sealable spout (5), the other of said first or second ends formed with an axially displaceable bottom (80), said bottom having a central opening therein;
an agitator (6) received within said vessel interior (2a), said agitator (6) comprised of a tubular rod (6b) which extends upwardly out of said interior through said opening in said bottom (80) and an agitator disk (6b) attached to said tubular rod, said tubular rod (6b) having an open, first end that defines a mouth and an open, second end that is encircled by said disk, said tubular rod (6b) axially displaceable within said vessel interior for mixing said bone cement components (A, B); a means for holding and introducing said liquid component (A) into said interior of said mixing vessel (2);
wherein said means for holding and introducing is joined to said mouth of said tubular rod prior to feeding said liquid contents (A) into said vessel (2) through said tubular rod (6b) whereby harmful emissions are prevented from escaping the vessel (2) during introduction of said liquid component (A) due to the vacuum present in said vessel (2), wherein said powder and liquid components (B, A) are mixed within said vessel interior (2a) under a continuous vacuum so that said harmful emissions caused from mixing are prevented from escaping said vessel (2), and wherein said mixed bone cement component (A, B) is pushed out of said sealable spout (5) through a displacement of said vessel bottom (80).

2. The apparatus of Claim 1, wherein said means for holding and introducing said liquid component (A) is operatively connected to the agitator rod (6b) in order to be used as a handle during mixing.

3. The apparatus of Claim 1 wherein said means for holding and introducing said liquid component (A) is comprised of a generally cylindrical container that is defined by a top, a bottom and an interior, said bottom having an opening therein which is in communication with said mouth of said tubular rod (6b), said top having a removably threaded cap (18) thereon for engagement with an ampoule (11) containing said liquid (A), said ampoule received within said container interior and resting on a means (13b) for breaking said ampoule, said breaking means (13b) anchored to said container interior above said opening in said container bottom, said cap having a reclosable opening (18d) therein and a pair of shoulders (18s) in contact with said ampoule, said shoulders (18s) for pushing said ampoule (11) against said breaking means (13b) through a downward threading of said cap (18), thereby breaking said ampoule (11) so that said liquid (A) is introduced into said tubular rod (6b) and into said mixing vessel (2).

4. A method for successively feeding in an arbitrary sequence batches of a liquid bone cement component (A) into a mixing vessel (2) maintained under vacuum for the preparation of said bone cement wherein said mixing vessel (2) is provided with a pre-determined amount of said powder component (B) of said cement, the method comprising the steps of providing a mixing vessel (2) which said vessel is defined by a cylindrical cylinder having an open interior (2a) with a spout (5) attached to one end of said cylinder, and having an axially displaceable bottom (80);
inserting a mixing agitator (6) within said spout so as to communicate with said vessel interior (2a), said agitator (6) comprised of a tubular rod (6b) having an agitator disk (6a) fixed on one end thereof, said other end being open and defining a mouth, said mouth being located axially above said spout of said vessel, said agitator (6) axially displaced such that said agitator disk (6a) can mix both of said bone cement components (A, B) together;
placing a container which has an open interior for receiving a glass ampoule (11) and a threadable cap (18) for pushing downwards on said ampoule, said interior including a means (13a) for breaking said ampoule (11) when said cap (18) pushes on said ampoule (11) thereby allowing said container to feed liquid (A) into said vessel (2) in said mouth of said tubular rod (6b), and operatively connecting container (9) and rod (6b) for using the container (9) as a handle during mixing. axially displacing said agitator (6) so as to mix said liquid and powder components (A, B) under vacuum, without allowing harmful emissions to escape said mixing vessel.
